# EUROPEAN PATENT APPLICATION

(11) **EP 4 781 941 A1**
(43) Date of publication of application: **29.07.2026**
(21) Application number: 26152026.6
(22) Date of filing: 15.01.2026
(51) Int. Cl.: A61B 34/20, A61B 17/00, A61B 5/053

(54) **SURGICAL REGISTRATION INSTRUMENT WITH MATERIAL CHARACTERIZATION CAPABILITIES**

(30) Priority: 22.01.2025 US 202563748107 P
(71) Applicant: Stryker European Operations Limited, Carrigtwohill, Co. Cork T45 HX08 (IE)
(72) Inventor: FAUL, Stephen, Rochestown, Cork, T12 H5CC (IE); MCKEE, Bruce, Ballycotton, Cork, P25 WV09 (IE); MORGAN, Jonathan Mark, Biscayne Park, FL, 33161 (US); BUCKLEY, Kevin, Bishopstown, Cork, T12EPY5 (IE); EUSTACE, David, Carrigrohane, Cork, T12 TDF3 (IE)
(74) Representative: Röthinger, Rainer

(57) **Abstract**

A surgical system includes a navigation system with a localizer and a surgical instrument having a body, a shaft with a distal tip for contacting material associated with an anatomy, and a tracking device detectable by the localizer. The shaft houses first and second conductors, insulated from each other, extending to the distal tip. One or more controllers are configured to transmit a variable-frequency signal across the conductors, measure impedance of the material based on the signal, and characterize the material type based on the measured impedance.

## Description

### CROSS-REFERENCE TO RELATED APPLICATIONS

The subject application claims priority to and all benefits of United States Provisional Patent App. No. 63/748,107, filed January 22, 2025, the entire contents of which are hereby incorporated by reference.

### BACKGROUND

Navigation systems assist users in precisely locating objects during surgery. For instance, navigation systems can assist surgeons in precisely placing surgical instruments relative to a patient's anatomy. Often, the instrument and the anatomy are tracked together with their relative movement shown on a display. To understand the location of the anatomy in the navigation coordinate space, a registration process is typically performed wherein a tracked pointer is used to touch the anatomy with a pointer tip. The registration process can be used to map pre-operative images of the anatomy to the real anatomy. Such registrations are often done by surgical guidance wherein a display presents landmark points that should be contacted by the probe. The surgeon then aims to place the pointer at the appropriate landmarks by viewing the display and by using their sense of touch and feel of the anatomy with the pointer. An input device, such as foot pedal, is then used to acquire the respective points.

Due to the biological complexity of surgical sites and the limitations of conventional pointers, typical anatomical registration techniques have several shortcomings. For example, inaccuracies may arise due to the pointer not contacting the proper surface/tissue for registration (e.g., the bone surface). There may be cartilage, soft tissue, or other objects that may disturb the pointer tip, causing inaccurate mapping. In other instances, the surgeon may wrongly believe the pointer tip is rested on the surface, when in fact the pointer tip may be temporarily lifted in air just above the surface. Conventional pointers are typically passive structures and are not provided with any sensing or controlling capabilities. Therefore, to a large degree, the navigation system must rely on the surgeon's judgment that the pointer tip is contacting the correct location and/or the correct tissue type. These various limitations can cause registration errors which can compromise the accuracy of the procedure.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided, comprising: a navigation system comprising a localizer; and a surgical instrument comprising: a body; a tracking device coupled to the body and being configured to be detected by the localizer of the navigation system; and a shaft coupled to the body and comprising a distal tip configured to contact a material associated with an anatomy; a first conductor extending along the shaft to the distal tip; and a second conductor being insulated from the first conductor and extending along the shaft to the distal tip; and one or more controllers coupled to the first and second conductors and being configured to: command, across the first and second conductors, transmission of a signal having a variable frequency; based on the signal, measure an impedance of the material as the distal tip is contacting the material; and characterize a type of the material based on the measured impedance.

According to a second aspect, a surgical instrument is provided, comprising: a body; a tracking device coupled to the body and being configured to be detected by a navigation system; a shaft coupled to the body and comprising a distal tip configured to contact a material associated with an anatomy; one or more controllers coupled to the body; a first conductor coupled to the one or more controllers and extending along the shaft to the distal tip; and a second conductor coupled to the one or more controllers, being insulated from the first conductor, and extending along the shaft to the distal tip; and wherein the one or more controllers are configured to: command, across the first and second conductors, transmission of a signal having a variable frequency; based on the signal, measure an impedance of the material as the distal tip is contacting the material; and characterize a type of the material based on the measured impedance.

According to a third aspect, a surgical instrument includes tracking device, a shaft coupled to the tracking device, a pair of electrodes, and a sensor. The shaft includes a distal tip. The pair of electrodes are located at the distal tip. The sensor is configured to detect a type of a tissue contacted by the distal tip.

According to a fourth aspect, a surgical instrument includes tracking device, a shaft coupled to the tracking device, a pair of electrodes, and a sensor. The shaft includes a distal tip. The pair of electrodes are located at the distal tip. The sensor is configured to detect whether a tissue contacted by the distal tip is bone or cartilage.

According to a fifth aspect, a surgical instrument includes a tracking device, a shaft coupled to the tracking device, a pair of electrodes, and a controller. The shaft includes a distal tip. The pair of electrodes are located at the distal tip. The controller is configured to transmit across the pair of electrodes a signal having a variable frequency, measure an impedance of the signal in response to the distal tip contacting an anatomy, and utilize the impedance to characterize a type of tissue of the anatomy.

According to a sixth aspect, a surgical navigation system is provided that includes a localizer; a probe comprising: a tracking device detectable by the localizer, a shaft coupled to the tracking device and including a distal tip configured to contact a tissue, and a pair of electrodes located at the distal tip; and a controller configured to: calculate impedance of the tissue contacted by the distal tip; characterize the tissue as bone based on the impedance; and enable the localizer to register a position of the probe upon determination that the characterized tissue is bone; and prevent the localizer from registering a position of the probe upon determination that the characterized tissue is not bone.

According to a seventh aspect, a surgical system is provided that includes a navigation system comprising a localizer; a surgical instrument comprising: a tracking device detectable by the localizer, a shaft coupled to the tracking device and including a distal tip configured to contact various tissues on an anatomical surface, and a pair of electrodes located at the distal tip; and a controller configured to: calculate impedances of the various tissue contacted by the distal tip; characterize the various tissues based on the impedances; and wherein the navigation system is configured to: utilize the localizer to register positions of the surgical instrument and corresponding tissue characterizations; generate a 3D surface representation of the anatomical surface including the tissue characterizations.

According to an eight aspect, a surgical instrument includes a body, a tracking device coupled to the body, a shaft coupled to the body and extending to a distal tip, a controller coupled to the body, and a pair of electrodes integrated into the shaft and extending from the controller to the distal tip, the controller is configured to implement a variable frequency sweep, within a specified range, across the pair of electrodes, identify an impedance of the material contacted by the distal tip based on the frequency sweep, and characterize a type of material based on the identified impedance.

Also provided are a method of operating any one or more of: the surgical system of any preceding aspect, the surgical instrument of any preceding aspect, the one or more controllers of any preceding aspect. Also provided are a non-transitory computer readable medium or computer program product, comprising instructions, which when executed by one or more processors, implement operation of: the surgical system, the surgical instrument, and/or the one or more controllers, of any aspect above. Any of the above aspects may be combined, in whole or in part.

Any of the above aspects may be combined with any of the following implementations. Any of the following implementations may be utilized in part, or in whole, with any of the above aspects. The implementations are:

The distal tip of the surgical instrument is configured to contact the material to facilitate acquisition of (potential) registration points pursuant to an anatomical registration procedure involving registration of the anatomy to the localizer of the navigation system. Each registration point is based on a position of the distal tip detected by the localizer. The one or more controllers are configured to evaluate the characterized material type to determine whether to acknowledge a position of the distal tip, or the respective registration point, during the anatomical registration procedure. The surgical instrument can contact one or more landmarks of the material to facilitate registration of the material to the navigation system. Registration can be imageless registration (without using medical images/models) or image-based registration to map intraoperative or preoperatively acquired medical images/models to the anatomy.

The material may be any type anatomical or non-anatomical material, or bone and non-bone material. If anatomical, the material may be of any naturally occurring object or material of the anatomy, including but not limited to: tissue, soft tissue, hard tissue, cortical bone, cancellous bone, osteophyte, cartilage, fat, muscle, tendon, ligament, blood vessel, blood, nerves, organic fluid, etc. If non-anatomical, the material may be of any non-naturally occurring object or material of the anatomy, including but not limited to: an implant or implant component, metal or metal fragments, a screw or a fastener, a tool checkpoint, a surgical tool, non-organic fluid, irrigation fluid, and the like. In some cases, the material can include gas, (ambient) air, or smoke proximate to a surface of the anatomy. The surgical instrument can be hand-held, mounted to a robotic arm, or mounted to an adjustable support arm. The surgical instrument can be hand-moved or hand-guided by a user when attached to the robotic arm or adjustable support arm. The surgical instrument (or body) can be held free-hand by a user (i.e., manually supported by the user against gravity).

The controller may be configured to acknowledge the position of the distal tip relative to the material, or respective registration point, in response to determining that the characterized material type is bone. The controller may automatically acknowledge the position of the distal tip relative to the material, or respective registration point, in response to determining the characterized material type is bone. The controller may be configured to acknowledge the position of the distal tip relative to the material, or respective registration point, in response to determining that the characterized material type is bone and upon receiving a user input. The user input may be a button coupled to the body of the surgical instrument. The user input may be a foot pedal coupled with the surgical navigation system. The controller may be configured to disregard the position of the distal tip relative to the material, or respective registration point, in response to determining that the characterized material type is non-bone material. Non-bone material comprises but is not limited to: cartilage, soft tissue, ligamentous tissue, tendon tissue, a blood vessel, gas, or air adjacent to the anatomy, metal material, implant material, a surgical tool material, and fluid material.

The surgical system can include a feedback device to provide feedback about characterized material. The feedback device can be audible, visual, haptic, or any combinations thereof. The feedback device can be part of the surgical instrument or provided elsewhere, such as a display of the navigation system. For example, the feedback can include a textual or verbal description of the material characterization. The feedback device can be provided on the surgical instrument, with the navigation system, or combinations thereof. The one or more controllers control the feedback device to generate first feedback in response to the characterized material type being bone and a second feedback in response to the characterized material type being non-bone material, wherein the second feedback is different from the first feedback. The one or more controllers can control the feedback device to generate the second feedback only if the non-bone material is specifically designated non-bone material, such as cartilage. The one or more controllers can control the feedback device to generate third feedback only if the non-bone material is other than cartilage (e.g., soft tissue or air), wherein the third feedback is different from the first and second feedback. The navigation system can include a display and the navigation system is configured to present, on the display, a graphical representation of the anatomy and the surgical instrument, a relative spatial relationship between the surgical instrument and the anatomy as determined by the localizer, the one or more of the registration points located on a surface of the of the graphical representation of the anatomy, and an indication of the material characterization associated with the one or more of the registration points.

The controller may be located on the body of the surgical instrument. The system may further include a console, wherein the controller is coupled to the console. The controller may be located remote from the surgical instrument. The controller may be coupled to the navigation system. The surgical instrument may be a probe, a drill, or a pointer. The variable frequency can be implemented, by the one or more controllers, as a frequency sweep. The frequency sweep can be across a specified range. The specified range can be defined from 6kHz to 100 kHz. The one or more controllers can perform the frequency sweep in response to detection of contact between the distal tip and the material or any other suitable condition, such as user input. The sweep can be performed any one or more number of times and can be performed in any order (high to low frequencies, or vice versa). In some cases, instead of a continuous frequency sweep, the variable frequency may be implemented as a discrete number of frequencies applied at discrete times.

The first conductor and/or the second material may be configured to contact the material. The distal tip may integrally extend from the shaft. The first conductor may extend along the shaft and terminate at a distal conductor end. The distal tip may circumferentially surround the discal conductor end. The first conductor may be an enamel coated copper wire. The second conductor may be integrally coupled to the shaft and terminate at the distal tip. The shaft of the surgical instrument may be comprised of steel.

### BRIEF DESCRIPTION OF THE DRAWINGS

Advantages of the present invention will be readily appreciated as the same becomes better understood by reference to the following detailed description when considered in connection with the accompanying drawings wherein:
FIG. 1 is a perspective view of a surgical system implementing various tracking systems, according to some implementations.
FIG. 2 is a schematic view of an example control system that can be used with the surgical system, according to some implementations.
FIG. 3 is a perspective view of a hand-held surgical instrument having a tracking device, according to some implementations.
FIG. 4 is a cross-sectional view of a shaft of the hand-held surgical instrument of FIG. 3, according to some implementations.
FIG. 5A is a perspective view of a distal tip of the shaft of FIG 3, according to some implementations.
FIG. 5B is a perspective view of the distal tip of the shaft of FIG 3, according to some implementations.
FIG. 6 is a perspective view illustrating an example operation of the surgical device to register an anatomy to the tracking system, according to some implementations.
FIG. 7 is a perspective view illustrating another example operation of the surgical instrument device, wherein the distal tip contacts a landmark of the anatomy, according to some implementations.

### DETAILED DESCRIPTION

### I. Example System Overview

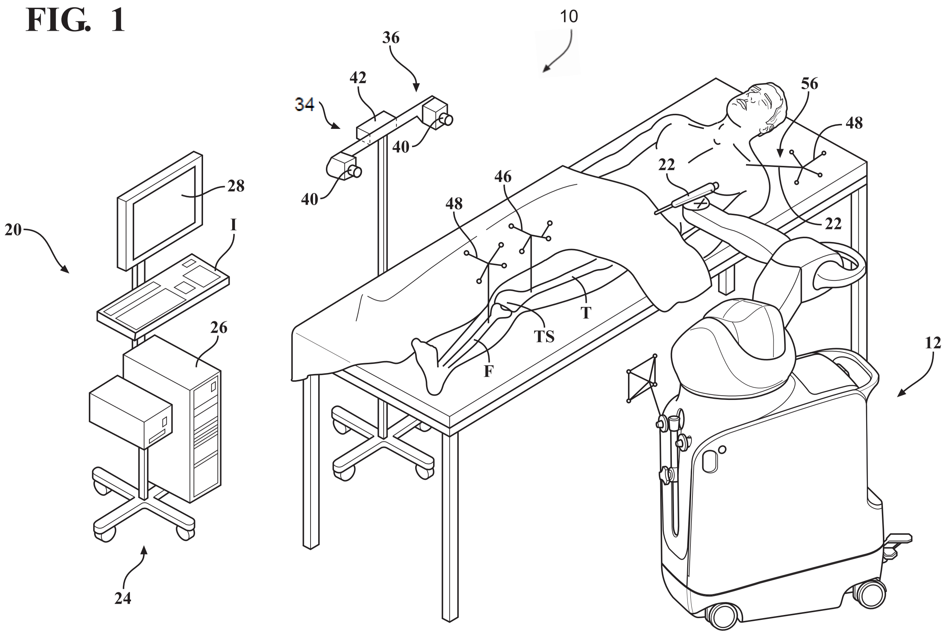

Referring to FIG. 1, a system 10 is provided. The system can be a surgical system 10 adapted for treating an anatomy or target site TS of a patient. The surgical system 10 is shown in a surgical setting such as an operating room of a medical facility. The surgical system 10 may be used to perform any intraoperative surgical procedure on a patient. Example surgical procedures include, but are not limited to: partial knee arthroplasty, total knee arthroplasty, total hip arthroplasty, shoulder arthroplasty, spinal procedures, ankle procedures, endoscopic procedures, cranial procedures, lesion removal procedures, arthroscopic procedures, arthroscopic resection procedures, soft tissue or ligament repair procedures, neurological procedures, ENT procedures, minimally invasive MIS procedures, or the like.

The target anatomy may be any part of the patient under operation, as an example, a bone is used herein. In the example shown in FIG. 1, the patient is undergoing a knee arthroplasty procedure in which material is removed from a femur F and/or a tibia T of a patient in preparation for receipt of an implant. However, it should be recognized that the surgical system 10 may be used to perform any suitable procedure in which material is removed from any suitable portion of a patient's anatomy, material is added to any suitable portion of the patient's anatomy (e.g., an implant, graft, etc.), and/or in which any other control of and/or visualization of a surgical tool is desired.

As will be described below, the techniques herein describe the use of the surgical system 10 in performing a procedure using a surgical instrument 56 to perform a frequency sweep to determine a material using impedance. Such techniques can be used for many purposes, such as for performing an anatomical registration process to register the target anatomy to the navigation system, or other purposes that may involve characterizing materials at the target site, such as identifying clinically relevant features or objects.

In the implementation shown, the surgical system 10 can include a manipulator 12 (e.g., surgical robot) and a navigation system 20. The navigation system 20 is set up to track movement of various objects in the operating room. Such objects include, for example, a surgical tool 22, a femur F of a patient, and a tibia T of the patient. The navigation system 20 can track these objects for purposes such as displaying their relative positions and orientations to the surgeon on a clinical application and, in some cases, for purposes of controlling or constraining movement of the surgical tool 22 relative to virtual cutting boundaries associated with the femur F and tibia T.

While the surgical system 10 is illustrated in FIG. 1 as including the surgical tool 22 attached to the manipulator 12, it should be recognized that the surgical system 10 may additionally or alternatively include one or more manually operated or hand-held surgical tools 22. As will be described in the subsequent sections below, the surgical tool 22 can be the surgical instrument 56 suitable for the characterizing material at the surgical site. For other examples, the surgical tool 22 may include a hand-held motorized saw, drill, bur, probe, robotized hand-held tool with actuators to move the surgical tool 22 relative to a hand-held grasping portion, or other suitable tool that may be held and operated by a surgeon. Any implementations described with reference to the use of the manipulator 12 may also apply to the use of a hand-held tool 22 with appropriate modifications. The surgical tool 22 may have working end or an energy applicator, such as a rotating bur, saw, router, reamer, impactor, electrical ablation device, cut guide, tool holder, probe, or the like. In other examples, the surgical tool 22 may be a camera tool, such as an endoscope, a laparoscope, an arthroscope, or a microscope. Any of the surgical tools 22 could be supported and moved by the manipulator 12 or mounted to an adjustable support arm.

The navigation system 20 can include one or more computer cart assemblies 24 that houses one or more navigation controllers 26. A navigation interface is in operative communication with the navigation controller 26. The navigation interface includes one or more displays 28 adjustably mounted to the computer cart assembly 24 or mounted to separate carts as shown. Input devices I such as a keyboard and mouse can be used to input information into the navigation controller 26 or otherwise select/control certain aspects of the navigation controller 26. Other input devices I are contemplated including a touch screen, a microphone for voice-activation input, an optical sensor for gesture input, and the like.

A clinical application can be displayed on one or more displays 28 of the navigation system 20. The clinical application assists a surgeon or staff in performing the surgical procedure. The clinical application can have a plurality of different screens related to the surgical procedure. Such screens can include a pre-operative planning screen, an operating room setup screen, an anatomical registration screen, an intra-operative planning screen, an anatomical preparation screen, or a post-operative evaluation screen, and the like. The clinical application can present a navigation guidance region that displays one or more of the surgical objects tracked by a localizer 34 of the navigation system 20.

The localizer 34 communicates with the navigation controller 26. In the implementation shown, the localizer 34 is an optical localizer and includes a camera unit 36. The camera unit 36 has a housing comprising an outer casing that houses one or more optical sensors 40. The optical sensors 40 can detect light signals, such as infrared (IR) signals and/or visible light signals. Camera unit 36 can be mounted on an adjustable arm to position the optical sensors 40 with a field-of-view of the below discussed trackers that, ideally, is free from obstructions. The camera unit 36 includes a camera controller 42 in communication with the optical sensors 40 to receive signals from the optical sensors 40. The camera controller 42 communicates with the navigation controller 26 through either a wired or wireless connection. In other implementations, the optical sensors 40 communicate directly with the navigation controller 26. Position and orientation signals and/or data are transmitted to the navigation controller 26 for purposes of tracking objects. The computer cart assembly 24, display 28, and camera unit 36 may be like those described in U.S. Patent No. 7,725,162 to Malackowski, et al. issued on May 25, 2010, entitled "Surgery System," the disclosure of which is hereby incorporated by reference. The navigation controller 26 can be a personal computer or laptop computer. Navigation controller 26 includes the displays 28, central processing unit (CPU) and/or other processors, memory (not shown), and storage (not shown). The navigation controller 26 is loaded with software that converts the signals received from the camera unit 36 into data representative of the position and orientation of the objects being tracked. The navigation controller 26 includes a navigation processor. It should be understood that the navigation processor could include one or more processors to control operation of the navigation controller 26. The processors can be any type of microprocessor or multi-processor system. The term processor is not intended to limit the scope of any implementation to a single processor.

Navigation system 20 is operable with a plurality of tracking devices 46, 48, also referred to herein as trackers. In the illustrated implementation, one tracker 46 can be firmly affixed to the femur F of the patient and another tracker 46 can be firmly affixed to the tibia T of the patient. Trackers 46 are firmly affixed to sections of bone in an implementation. For example, trackers 46 may be attached to the femur F and tibia T in the manner shown in U.S. Patent No. 7,725,162 to Malackowski, et al. issued on May 25, 2010, entitled "Surgery System," the disclosure of which is hereby incorporated by reference. Trackers 46, 48 may also be mounted like those shown in U.S. Patent Application No. 14/156,856, filed on January 16, 2014, entitled, "Navigation Systems and Methods for Indicating and Reducing Line-of-Sight Errors," hereby incorporated by reference herein. The trackers 46, 48 may be mounted to other tissue types or parts of the anatomy. One or more tool trackers 48 can be coupled to the manipulator 12, the end effector of the manipulator 12, or to the base of the manipulator 12. Tool trackers 48 can also be attached to any of the hand-held tools 22 including the surgical instrument 56 at any suitable location. Any of these objects can be referred to as surgical tools 22. The tool tracker 48 can be integrated into the surgical tool 22 during manufacture or may be separately mounted to the surgical tool 22 in preparation for surgical procedures.

In one implementation, optical sensors 40 of the localizer 34 receive light signals from the trackers 46, 48. Some of the trackers 46, 48 may include passive markers. For example, the tracker can have at least three passive tracking elements or markers (e.g., reflectors) for transmitting light signals (e.g., reflecting light emitted from the camera unit 36) to the optical sensors 40. In other implementations, some, or all of the trackers 46, 48 may include active tracking markers. The active markers can be, for example, light emitting diodes transmitting light, such as infrared light. Active and passive arrangements are possible. The camera unit 36 receives optical signals from the trackers 46, 48 and outputs to the navigation controller 26 signals relating to the position of the tracking markers of the trackers 46, 48 relative to the localizer 34. Based on the optical signals received, navigation controller 26 generates data indicating the relative positions and orientations of the trackers 46, 48 relative to the localizer 34. These relative positions can be displayed on the clinical application as graphical representations for surgical guidance.

Furthermore, in some examples, the navigation system 20 can additionally or alternatively implement the localizer 34 as a vision tracking system. The vision-based localizer 34 includes a vision or video camera coupled to the navigation controller 26. The vision camera can be the one or more of the optical sensors 40. The vision camera facilitates acquisition of 2D and/or 3D machine-vision images or view of structural features that define trackable features such that tracked states of the objects are communicated to (or interpreted by) the navigation controller 26 based on the machine-vision images or view. The machine vision system can be integrated into the camera unit 36, optionally in combination with infrared sensors. The machine vision system can create depth maps and can detect objects with or without trackers. The machine vision system can detect patterns, shapes, colors, computer-codes, tracking geometries, and the like.

Additionally, or alternatively, the navigation system 20 and/or the localizer 34 can employ radio frequency (RF) based tracking. For example, the navigation system 20 may comprise an RF transceiver coupled to the navigation controller 26. Here, the trackers 46, 48 may comprise RF emitters or transponders, which may be passive or may be actively energized. The RF transceiver transmits an RF tracking signal, and the RF emitters respond with RF signals such that tracked states are communicated to (or interpreted by) the navigation controller 26. The RF signals may be of any suitable frequency. The RF transceiver may be positioned at any suitable location to track the objects using RF signals effectively. Furthermore, examples of RF-based navigation systems may have structural configurations that are different than the navigation system 20 illustrated throughout the drawings.

Additionally, or alternatively, the navigation system 20 and/or localizer 34 can employ aspects of electromagnetic (EM) tracking. For example, the navigation system 20 may comprise an EM transceiver coupled to the navigation controller 26. Here, the trackers 46, 48 may comprise EM components attached thereto (e.g., various types of magnetic trackers, electromagnetic trackers, inductive trackers, and the like), which may be passive or may be actively energized. The EM transceiver generates an EM field, and the EM components respond with EM signals such that tracked states are communicated to (or interpreted by) the navigation controller 26. The navigation controller 26 may analyze the received EM signals to associate relative states thereto. Here too, examples of EM-based navigation systems may have structural configurations that are different than the navigation system 20 illustrated throughout the drawings.

In other examples, the navigation system 20 and/or the localizer 34 could be based on one or more other types of tracking systems. For example, an ultrasound-based tracking system coupled to the navigation controller 26 could be provided to facilitate acquiring ultrasound images of markers that define trackable features on the tracked objects such that tracked states are communicated to (or interpreted by) the navigation controller 26 based on the ultrasound images. By way of further example, a fluoroscopy-based imaging system (e.g., a C-arm) coupled to the navigation controller 26 could be provided to facilitate acquiring X-ray images of radio-opaque markers that define trackable features such that tracked states are communicated to (or interpreted by) the navigation controller 26 based on the X-ray images. The shape of the trackers 46, 48 can also be of a geometry that can be identified in X-ray imaging to assist in registration.

Several types of tracking and/or imaging systems could define the localizer 34 and/or form a part of the navigation system 20 without departing from the scope of the present disclosure. Furthermore, the navigation system 20 and/or localizer 34 may have other suitable components or structure not specifically recited herein, and the various techniques, methods, and/or components described herein with respect to the navigation system 20 shown throughout the drawings may be implemented or provided for any of the other examples of the navigation system 20 described herein. For example, the navigation system 20 may utilize solely inertial tracking and/or combinations of different tracking techniques, sensors, and the like. Any of the described tracking methods can be included in the trackers 46, 48. Other configurations are contemplated.

Based on the position and orientation of the trackers 46, 48 and previously loaded data, navigation controller 26 can determine the position and/or the orientation of the surgical tool 22 relative to the tissue against which the working end is to be applied. Based on the position and/or orientation of the surgical tool, the navigation controller 26 may define a virtual boundary. For example, the anatomy can be registered and defined as a virtual boundary. In implementations, a surgical plan may be generated.

In one implementation, the manipulator 12 is controlled to stay within the virtual boundary. The virtual boundary may be a virtual cutting boundary which defines the material of the anatomy (e.g., the femur F and tibia T) to be removed by the surgical tool 22. For example, each of the femur F and tibia T may have a target volume of material that is to be removed by the working end of the surgical tool 22. The target volumes are defined by one or more virtual cutting boundaries. The virtual cutting boundaries define the surfaces of the bone that should remain after the procedure. The navigation system 20 tracks and controls the surgical tool 22 to ensure that the working end, e.g., the surgical bur, removes the target volume of material and does not extend beyond the virtual cutting boundary, as disclosed in U.S. Patent No. 9,119,655, entitled, "Surgical Manipulator Capable of Controlling a Surgical Instrument in Multiple Modes," the disclosure of which is hereby incorporated by reference, or as disclosed in U.S. Patent No. 8,010,180, entitled, "Haptic Guidance System and Method", the disclosure of which is hereby incorporated by reference.

The virtual cutting boundary may be defined within a virtual model of the anatomy (e.g., the femur F and tibia T), or separately from the virtual model. The virtual cutting boundary may be represented as a mesh surface, constructive solid geometry (CSG), voxels, or using other boundary representation techniques. The surgical tool 22 may be used to cut away material from the femur F and tibia T to receive an implant. The surgical implants may include unicompartmental, bicompartmental, or total knee implants as shown in U.S. Patent No. 9,381,085, entitled, "Prosthetic Implant and Method of Implantation," the disclosure of which is hereby incorporated by reference. Other implants, such as hip implants, shoulder implants, spine implants, and the like are also contemplated. The focus of the description on knee implants is provided as one example. These concepts can be equally applied to other types of surgical procedures, including those performed without placing implants.

The navigation controller 26 also generates image signals that indicate the relative position of the working end to the tissue. These images can be presented on the displays 28 by the clinical application to allow the surgeon and staff to view the relative position of the working end to the target site TS.

Before or during the procedure, images of the femur F and tibia T may be generated (or of other portions of the anatomy in other implementations). The images can be stored as two-dimensional or three-dimensional patient image data in a computer-readable storage device, such as memory (M) within the navigation system 20. The patient image data may be based on X-ray scans or computed tomography (CT) scans of the patient's anatomy. The patient image data may then be used to generate two-dimensional images or three-dimensional models of the patient's anatomy. The pre-operative data and models may be used for purposes of surgical planning purposes and intraoperative guidance. For example, the surgical plan may be planned relative to the virtual model. The virtual model and surgical plan can then be registered to the anatomy using any appropriate registration technique, such as pointer registration, imageless registration, or the like.

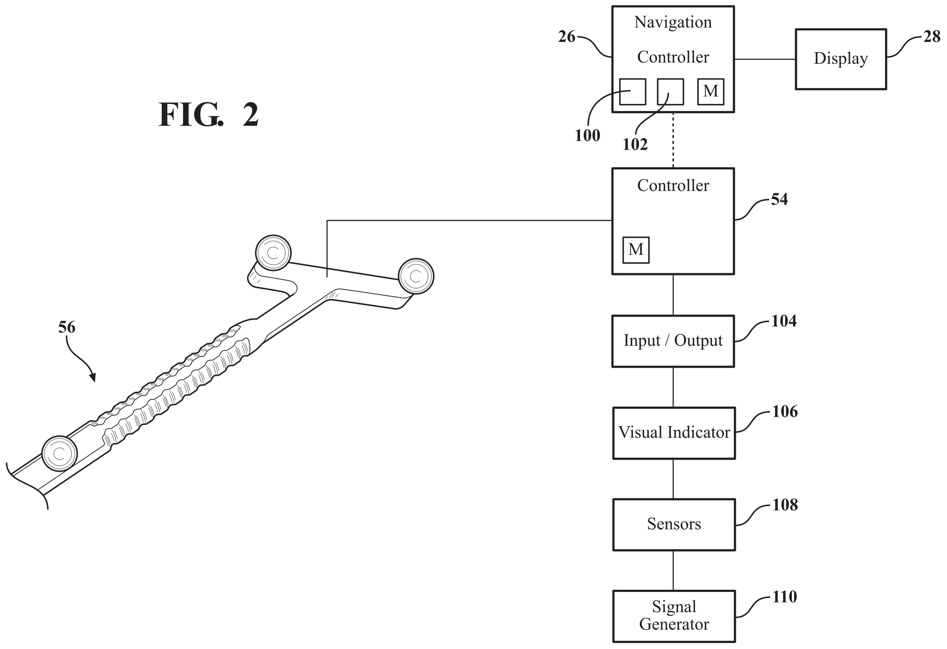

FIG. 2 illustrates a schematic view of an example control system that can be used with the surgical system 10. A localization engine 100 is a software module that can be considered part of the navigation system 20. Components of the localization engine 100 run on navigation controller 26. Localization engine 100 receives as inputs the signals from the localizer 34 and, in some implementations, signals from the trackers 46, 48. Based on these signals, localization engine 100 can determine the pose of each tracker coordinate system in the localizer coordinate system. The localization engine 100 forwards the signals representative of the poses of trackers 46, 48 to a coordinate transformer 102.

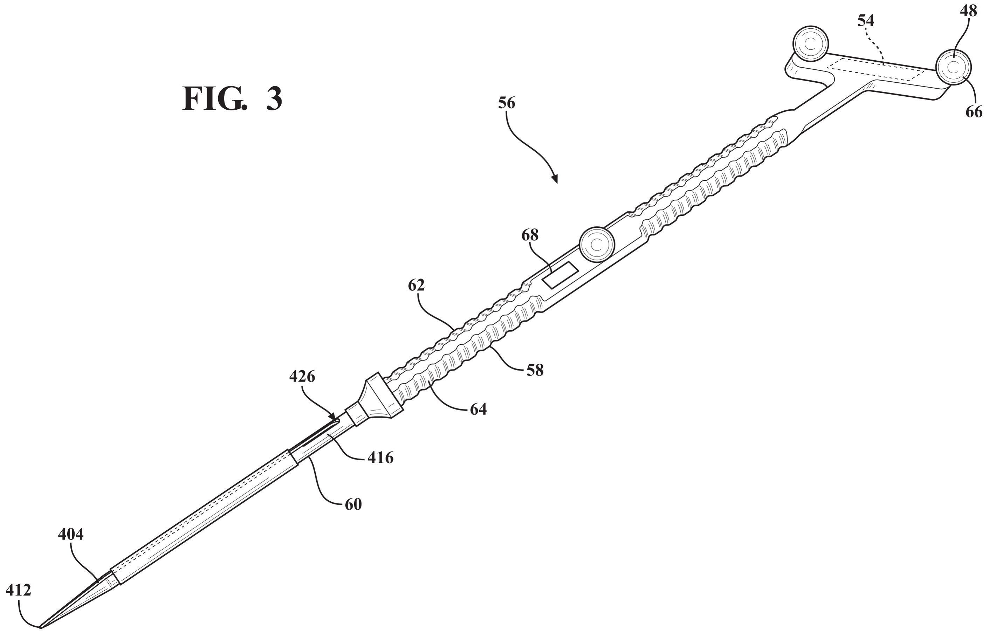

Coordinate transformer 102 is a navigation system software module that runs on navigation controller 26. Coordinate transformer 102 references the data that defines the relationship between the images of the patient and the anatomy trackers 46. Coordinate transformer 102 can also store the data indicating the pose of the working end of the surgical instrument 56 relative to a tool tracker 48. During the procedure, the coordinate transformer 102 receives the data indicating the relative poses of the trackers 46, 48 to the localizer 34. Based on these data, the previously loaded data, and data from the surgical instrument 56, the coordinate transformer 102 generates data indicating the position and orientation of the distal tip 412 (FIG. 3) of the surgical instrument 56 relative to the tissue (e.g., bone) against which the working end is applied. Image signals representative of these data is forwarded to displays 28 enabling the surgeon and staff to view this information.

In implementations, the surgical system 10 is utilized to transform relevant coordinates into a bone coordinate system so that the position and/or orientation of the surgical tool 22 can be tracked relative to the position and orientation of the bone (e.g., the femur model) and/or the position and orientation of the volume of material to be treated by the surgical tool 22. The relative positions and/or orientations of these objects can also be represented on the displays 28 to enhance the user's visualization before, during, and/or after surgery.

While the example surgical system 10 has been described with reference to the Figures, the surgical system 10 is not intended to be limited to what is specifically shown and described. Other systems are contemplated without departing from the scope of the disclosure.

### II. Surgical Probe Configurations

With reference to FIGS. 2-5B, the surgical instrument 56 for the characterization of materials associated with an anatomy is illustrated. The surgical instrument 56 is configured to contact material at the surgical site, such as bone, soft tissue, cartilage, or foreign body objects. Using the configuration and techniques described herein, the surgical instrument 56 and/or the surgical system 10 can characterize the material. The surgical instrument 56 can be hand-held, mounted to a robotic arm of the manipulator 12, or mounted to an adjustable support arm.

The surgical instrument 56 may be a pointer/probe, a drill, or other instrument that includes a shaft and tip. The surgical instrument 56 includes a body 58, a tracker 48, a shaft 60 coupled to the body, a first conductor 402, a second conductor 404, and one or more controllers 54. In examples, the surgical instrument 56 may include one or more buttons 68 configured to suit a specific operation of the surgical instrument.

The controller 54 can include an input and output device 104. The input and output device 104 sends and receives communication from the navigation controller 26. The input and output device 104 can also communicate with other controllers and devices of the surgical system 10. For example, the input and output device 104 may communicate with the input device I of the computer cart assembly 24 or the display 28. Other input and output devices 104 include buttons, microphones, touchpads, cameras, and/or external hard drives. The input and output devices 104 may communicate using a wired connection and/or a wireless communication system such as WiFi, Bluetooth, Infrared, or the like. In some implementations, the localization engine 100 may run on a controller 54 of the surgical instrument. Some, or all, of the capabilities described with reference to surgical instrument controller 54 can be equally implemented on the navigation controller 26.

The surgical instrument 56 can include one or more sensors 108. The sensors 108 may assist the navigation system 20 in determining an accurate orientation and position of the instrument. Additionally, the sensors 108 may assist a user in the use of the surgical instrument 56. As examples, the sensors 108 may include force sensors that may measure the amount of force applied to the surgical instrument 56, tactile sensors to detect textures and touch, position sensors to detect movement, pressure sensors to measure pressure on tissues, temperature sensors, optical sensors, and/or vibration sensors. In implementations, the sensors 108 can include an impedance sensor configured to calculate an impedance of a material contacting the distal tip 412 of the surgical instrument 56, as described in greater detail below.

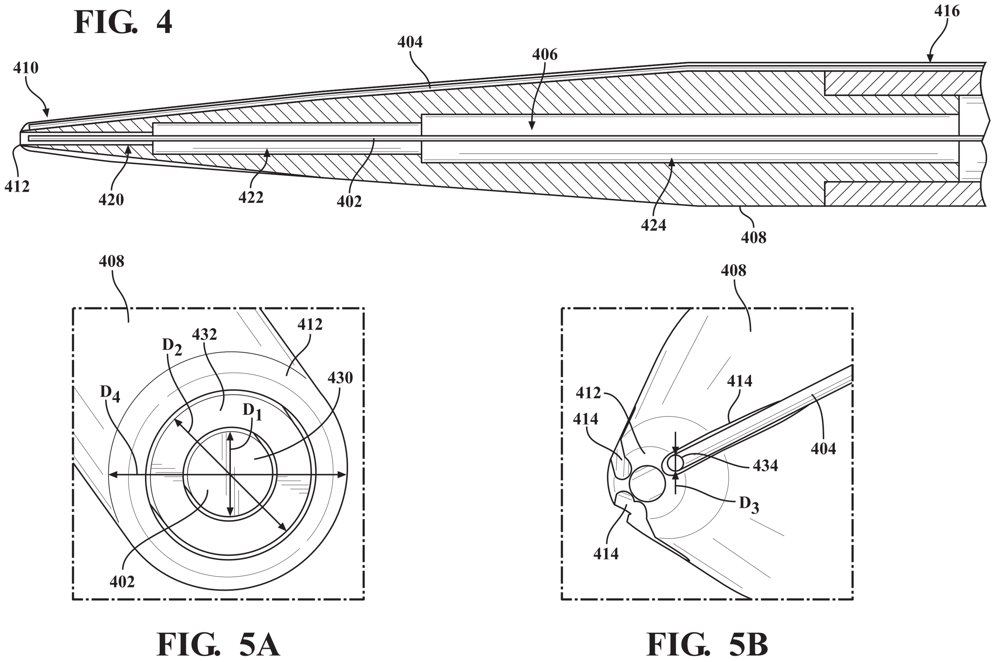

The surgical instrument includes a signal generator 110. The signal generator 110 outputs a signal through a first conductor 402 (FIG. 4) and inputs a return signal through a second conductor 404 (FIG. 4). The signal may be a varying AC signal. In implementations, the signal generator 110 may output a signal through the second conductor 404 and receive a signal input through the first conductor 402. It should also be understood that the controller 54, input and output devices 104, and other sensors 108 may cause the frequency sweep across the first conductor 402 and second conductor 404. The controller 54 of the surgical instrument 56 can cause a signal generator 110 to generate a signal of one or more select frequencies across the distal tip 412 of the surgical instrument 56. Select frequencies can be for example between 6kHZ to 100kHz. In one example, the signal is of variable frequency. In other examples, the signal is implemented as a frequency sweep. In examples, the frequency sweep may be between 6kHz and 100kHz. However, the controller may sweep between any number of frequencies or frequency ranges, for example 0kHz to 50kHz, 0kHz to 200kHz, 6kHz to 100kHz, or the like. Frequencies can be incremented in any suitable manner, such as linearly or logarithmically. Signal generator 110 communicates data in reference to the output and return signal to the controller 54. The data includes the voltage, current, frequency, and impedance of the signal. In implementation the signal generator 110 may communicate such data to the navigation controller 26. The one or more controllers can generate complex impedance profiles, Nyquist plots, Bode plots, or other data profiles to determine how the material electrical properties change with frequency. The controller(s) can include any suitable technology to analyze impedance, such as a function generator, impedance analyzer, amplifiers, and the like.

Responsive to transmission of the signal, the controller 54 measures the impedance of the material in response to the distal tip 412 contacting the material and utilizes the frequency and measured impedance to characterize a type of material the distal tip 412 is contacting. It should be understood that any part of such process may be conducted by a controller 54 positioned remote from the surgical instrument 56, including the navigation controller 26 or a controller or on the console, as described above. The material 602 is a part of the anatomy for registration to the navigation system 20. The anatomy may be a part of a surgical object 600 comprised of landmarks. For example, the surgical object 600 may have landmarks including checkpoints, bone, soft tissue, hard tissue, cartilage, implants, or the like. The characterized type of material of such landmarks may be bone, tissue, metals, cartilage, air, ligaments, implants, tools, checkpoint, or any other organic or inorganic material, fluid, or substance found in or around a body. The checkpoint can be screwed into the bone and can include a divot for placement of the distal tip 412. Implants can be primary implants that pre-existed in the target site during a revision procedure or implants that have recently been placed in a primary procedure.

Upon contact of the distal tip 412 with the material, the return signal may include data including a voltage, current, frequency. Using the data, the controller 54 may determine an impedance of the material. Data from the surgical instrument 56 can include data relating to the type of material the distal tip 412 of the surgical instrument 56 is applied. In implementation the coordinate transformer 102 will only generate the position if the material is a tissue such as bone. As such, if the surgical instrument 56 is intended to contact bone, and the surgical instrument 56 is detected to contact soft tissue, cartilage, air, metal, or other undesired material, the coordinate transformer 102 of the navigation controller 26 may be configured to not register the position of the surgical instrument 56 relative to the tissue.

The body 58 of the surgical instrument is shaped to allow a user to handle the surgical instrument 56. The body 58 can include a handle portion 62 comprised of a series of grooves and bumps 64 to help maintain a grip and reduce any slipping or mishandling. As an example, a user may manipulate the surgical instrument by holding the body of the instrument. The tracker 48, as described above, may be integrated into the body 58 of the surgical instrument 56. The tracker 48 integrated with the body 58 may include passive or active markers 66 to interact with the localizer 34 in order to register the position of the surgical instrument 56 with the surgical navigation system 20. The controller 54 may be coupled to the body 58 of the surgical instrument. In some examples, the controller 54 may be positioned on a console (not shown) of the surgical system 10, on the surgical navigation system 20, or otherwise remote from the surgical instrument 56.

Extending from the body 58 of the surgical instrument 56 opposite the tracker 48 is the shaft 60. The shaft 60 may include an outside surface 408 encircling a hollow central portion 406. However, it should be understood that the shaft 60 may be of any shape having an outside surface 408 and central portion 406. A distal end 410 of the shaft 60 opposite the body 58 of the surgical instrument tapers to a distal tip 412, wherein the central portion 406 extends from the body 58 to the distal end 410 of the shaft 60. The central portion 406 may have a diameter between .5mm and 2mm. The central hollow portion 406 may comprise a distal end bore 420, a proximal end bore 424, and a central bore 422 between the distal end bore 420 and the proximal end bore 424. The distal end bore 420 has a diameter less than the central bore 422. The central bore 422 has a diameter less than the proximal end bore 424. The distal end bore 420, the central bore 422, and the proximal end bore 424 are integrated as to extend from the body 58 to the distal tip 412. A central opening 426 extending through the outside surface 408 to the central portion 406 may be positioned along the shaft 60 between the distal end 410 and the body 58.

A series of grooves 414 may extend along the outside surface 408 and ending at the distal tip 412. In examples there is one groove 414. In other examples, there are any number of grooves 414. The grooves 414 may extend from the body 58 of the surgical instrument 56 or may start at a central position 416 between the distal tip 412 and the body 58. The groove may start at the central opening 426 and extend to the distal tip 412. Within the grooves 414, various wires and fibers may extend along the shaft 60, ending at the distal tip 412 of the shaft. Such wires may include sensors, lights, cameras, or other indicators. The shaft 60 may be comprised of steel, aluminum, silver, copper, or any other suitable material or metal. In some examples, the shaft 60 further includes impedance checkpoints at a set distance, where the impedance along the shaft is known. In such examples, the signal comprising a frequency sweep can be used to check for accuracy.

In one implementation one or more fibers are held within grooves 414 of the distal tip 412. These fibers could be used to transmit incident light to the tissue surface. The reflected light could return to an optical detector through the same or one of the other fibers. The light returned through these fibers could be analyzed to determine by optical characterization whether the tissue at the fiber's distal tip is cartilage or bone. This describes an optical implementation of determining bone surface position used in place of or additional to the electrical implementation.

The first conductor 402 may be an electrode wire extending along the shaft 60, terminating at a distal conductor end 430. The distal conductor end 430 is generally flush with the distal tip 412 of the shaft 60 such that upon the distal tip 412 contacting a material, the distal conductor end 430 of the first conductor 402 contacts the material. The first conductor 402 may include a second end that is coupled to the signal generator 110 and/or the controller 54. In examples, the outside surface 408 of the shaft 60 circumferentially surrounds the distal conductor end 430 of the first conductor 402. The diameter of the distal tip D4 may be 1mm. In examples, the diameter of the distal tip D4 may be between .75mm and 1.5mm. However, it should be understood that the diameter of the distal tip D4 may be any distance required to contact a material associated with an anatomy. A diameter of the distal conductor end 430 may be .5mm. In examples the diameter of the distal conductor end D1 is between 25 and 75 percent the diameter of the distal tip D4.

An insulating layer 432 is positioned between the first conductor 402 and the shaft 60. The insulating layer 432 may be integral with the first conductor 402. In some examples, the insulating layer 432 is integral with the shaft 60. The insulating layer 432 is generally flush with the distal tip 412 of the shaft 60 and the distal conductor end 430. The diameter of the insulating layer D2 is greater than the diameter of the first conductor D1 and less than the diameter of the distal tip D4. In examples, the first conductor 402 is a copper wire. The copper wire may include an enamel coating, wherein the enamel coating acts as the insulating layer 432.

The second conductor 404 may be an electrode extending along the outside surface 408 of the shaft 60 within a groove 414 or may be integrally coupled to the shaft 60. The second conductor 404 may be a wire that extends from the distal tip 412 of the shaft to the signal generator 110 or controller 54. The second conductor 404 may be an electrode wire soldered to the shaft 60. In some examples, the shaft 60 acts as the second conductor 404. In such examples, the distal tip 412 of the shaft 60 acts as the second conductor 404 upon contact with the material. In other examples the second conductor 404 extends from the signal generator 110 or controller 54 and through the groove 414 within the outer surface 408 of the shaft 60. The second conductor may extend through the central opening 426 to couple to the signal generator 110 or controller 54. The second conductor 404 includes a second conductor end 434 that is generally flush with the distal tip 412 of the shaft 60, ensuring contact with the material upon the distal tip 412 contacting the material. In such examples, the diameter of the second conductor end D3 can be between .15mm and .5mm. The first and second conductor 402, 404 may be steel, aluminum, silver, copper, or any other suitable material or metal.

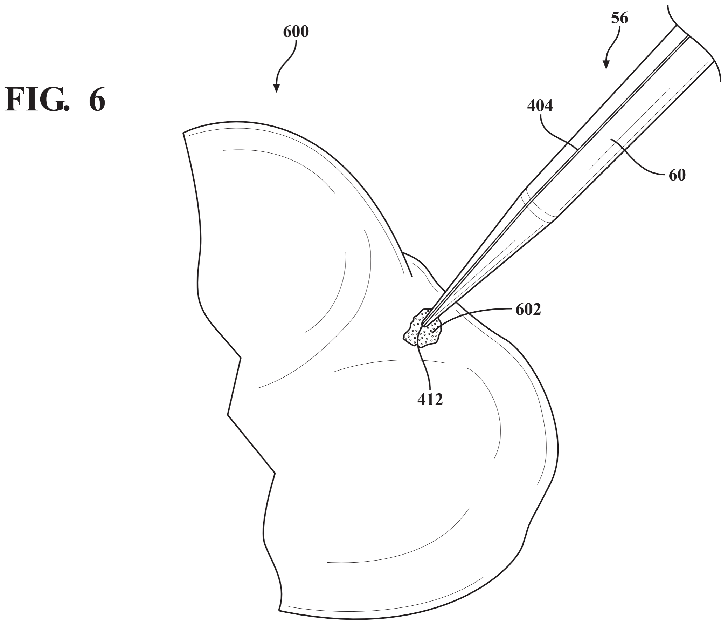

The distance between the first conductor 402, the second conductor 404, and the insulating layer 432 between the first conductor 402 and second conductor 404 is known to measure impedance. In implementations, the first conductor 402 is an enamel coated copper wire with a known diameter and acts as pole one. The second conductor 404 is soldered to the distal tip 412, causing the distal tip 412 of the shaft 60 to act as a steel second conductor 404. The second conductor 404 is pole two. As the distal tip 412 is pressed against a contact material 602 (FIG. 6), the signal generator 110 conducts the frequency sweep and bioimpedance is then measured by controller 54 between pole one and pole two.

The surgical system can include a feedback device to provide feedback about characterized material. The feedback device can be audible, visual, haptic, or any combinations thereof. The feedback device can be part of the surgical instrument or provided elsewhere, such as a display of the navigation system. For example, the feedback can include a textual or verbal description of the material characterization. In some cases, the surgical instrument may include a visual indicator. In some cases, the surgical instrument 56 may include a visual interface or indicator 106. The visual interface or indicator 106 may include optical emitters or a screen/display. The controller 54 may cause the visual interface to display a notification or alert based on an action of the user and/or the type of material that the distal tip 412 of the surgical instrument 56 is applied. As an example, the visual indicator may flash a color upon contact with a material. As another example, the visual interface or indicator 106 may give an error message upon incorrect use by a user. Although visual interface or indicators 106 have been contemplated, it should be understood that audible notifications and alarms may also be emitted from the surgical instrument 56.

### III. Material Characterization

With reference to FIGS. 6-7, examples of the controller 54 characterizing material 602 of the surgical object 600 upon contact of the distal tip 412 to a part of the surgical object 600 are illustrated. Upon characterizing a contacted material 602, the registration of the anatomy is facilitated by the controller 54. In some examples, as the surgical instrument 56 is moved to contact the surgical object 600, the surgical instrument 56 may contact various types of materials, as described above. In such examples, only certain contact materials 602 are desired for registration. For instance, the surgical instrument 56 may move through cartilage 606 in order to contact bone 608. In the implementation described herein, bone 608 is the desired material for registration of the anatomy. The distal tip 412 of the surgical instrument 56 is configured to contact the material to facilitate acquisition of (potential) registration points pursuant to the anatomical registration procedure involving registration of the anatomy to the localizer of the navigation system. Each potential registration point is based on a position of the distal tip detected by the localizer. As will be described, these registration points may be acknowledged/disregarded depending on the characterized material associated with the respective points. With respect to contacting the material, the distal tip 412 position and registration point are used interchangeably herein based on the understanding that the registration point may or may not be used for registration in view of the characterized material.

FIG. 6 illustrates an example of the surgical instrument 56 interacting with a bone. As the surgical instrument 56 moves to contact multiple types of material 602, the controller transmits the signal of variable frequency, as described above, and determines the impedance of the contacted material 602. Based on the measured impedance and known frequencies, the type of material can be characterized by the controller 54. As an illustrative example, the controller 54 may send a frequency sweep and obtain a current and voltage across a material. The controller 54 may calculate the impedance of the material based on the measured values. From the impedance, the controller may compare the impedance to known impedances of material. The controller 26 may then characterize the material. Consequently, in some examples, the controller 54 can determine when the surgical instrument is in contact with the desired material and register the point to the navigation system 20.

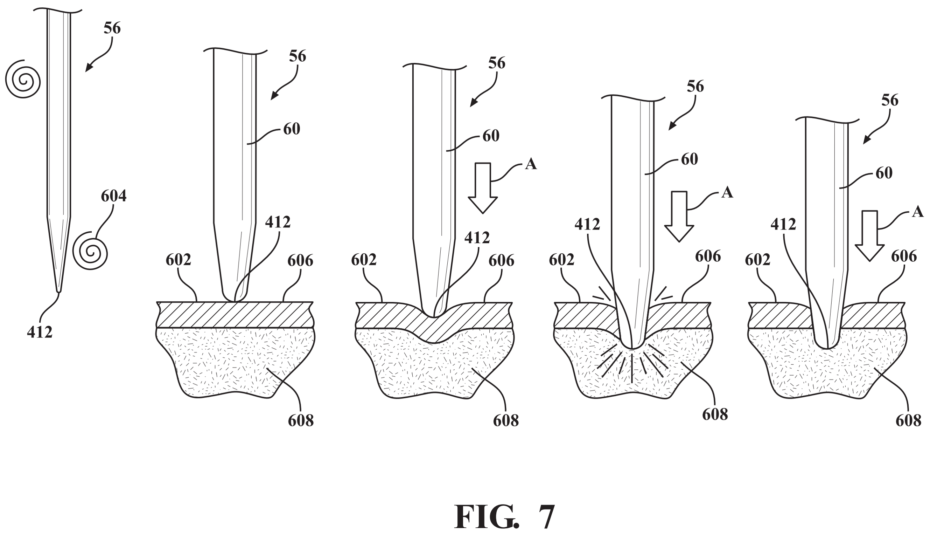

As depicted in FIG. 7, the distal tip 412 of the surgical instrument 56 first contacts air 604. As the surgical instrument 56 moves in the direction of arrow A, the distal tip 412 contacts cartilage 606. The distal tip 412 may pop or push through the cartilage 606 to contact bone 608. For registration of the anatomy to the navigation system 20, the controller 54 facilitates registration of the surgical instrument's 56 position, as described above, only upon contact with a desired material. Upon contact with the desired material, the surgical system 10 may use the tracker 48 positioned on the surgical instrument 56 to determine the position of the distal tip 412 of the surgical instrument 56 in reference to a surgical object 600 made up of the materials, thereby mapping the position of the surgical object 600 using registration points. In some examples, the desired material is bone. In other examples, the desired material may be a checkpoint or metal.

To facilitate the characterization of the contact material 602, the controller 54 is configured to command the transmission of the signal having a variable frequency across the first and second conductors 402, 404. As described above, the signal may be a frequency between 0 and 100kHz. The controller 54 is configured to calculate an impedance value based on a measured current and voltage at a frequency. The signal may be an AC signal and the impedance may be a complex impedance. Using the impedance, the controller 54 is configured to determine a characterization of the contact material 602. For example, the controller 54 may determine that the distal tip 412 is contacting air, cartilage, or bone. In some examples, the controller 54 may use a ratio of the impedance at multiple frequencies to determine the characterization of the material. The controller 54 may determine a ratio of impedance. In examples, the ration of impedance may be a ratio between any number of the impedances measured using the frequency sweep. The ratio may be between the impedance at two frequencies, or a larger amount of frequencies. The controller 54 may determine a type of tissue at the contact surface based upon the ratio of impedance. The surgical navigation system 20 is configured to register a position of the surgical instrument 56 upon a determination of a characterized type of contact material 602 or tissue.

Upon the characterization of the contact material 602, the controller 54 is configured to evaluate the characterized material type to determine whether or not to acknowledge the position of the surgical instrument 56 relative to the contact material 602 in order to register the anatomy to the navigation system 20. In some examples, upon a determination that the material is bone, the controller 54 is configured to acknowledge the position of the distal tip 412 (or respective registration point) relative to the bone 608. The controller 54 may be configured to acknowledge the position of the distal tip 412 (or respective registration point) automatically upon the determination that the material is bone 608. In some examples, the controller 54 acknowledges the position of the distal tip 412 (or respective registration point) only upon both a determination that the contact material 602 is bone 608 and a user input. A user input may be voice activation, the button 68 positioned on the surgical instrument 56, a foot pedal coupled to the surgical navigation system 20, a gesture given by the user, or other similar input.

Upon a determination that the characterized material type is cartilage 606, the controller 54 may be configured to disregard the position of the distal tip 412 relative to the contacted material 602. As such, in an example wherein the user contacts cartilage 606, the respective registration point may not be registered to the surgical navigation system 20. In some examples, the position of the distal tip 412 relative to the contact material 602 is disregarded when the distal tip 412 contacts cartilage and a user input. In some examples, a user input may override the determination that the characterized material type is cartilage and register the position of the distal tip 412 relative to the contact material 602 in order to register the anatomy to the surgical navigation system. In some examples, the controller 54 may be configured to disregard the position of the distal tip 412 relative to the contact material 602 (or respective registration point) if the material type is determined to not be bone 608.

Upon a determination of the type of contact material 602, the controller 54 may cause a notification or alarm to be displayed on the display 28 of the surgical system 10 or to the visual interface or indicator 106 positioned on the surgical instrument 56. The notification or alarm may be textual in nature, an audible alarm, or a set of colors. For example, the controller 54 may cause the visual interface or indicator 106 to light a first color for bone, a second color for cartilage, and a third color for air and/or soft tissue. The visual interface or indicator 106 may be a fiber positioned within a groove 414 of the shaft 60 or be positioned at any location on the surgical instrument 56.

As another illustrative example, a user used the surgical instrument 56 to press into the contact material 602 of the surgical object 600. The controller 54 transmits the signal of variable frequency and receives an impedance value. Using the frequency and impedance, the controller characterizes the contact material 602 to be cartilage 604. Upon this determination, the controller disregards an instruction to send the position of the distal tip 412 relative to the material 602. An instruction to the controller 54 may be preprogramed, sent by the navigation controller, sent by a user input, or some other form of instruction. In some examples, the controller 54 may send the position of the distal tip 412 relative to the material 602 despite the characterization of the contact material 602 as cartilage upon receiving a user input.

In another example, the controller 54 preforms the frequency sweep upon a user contacting a material and characterizes the contact material 602 as bone 608. In such examples, the controller 54 may have an instruction to automatically send the position of the distal tip 412 relative to the material to the navigation system 20. In similar examples, the controller 54 may only send the position of the distal tip 412 relative to the material to the navigation system 20 upon both the characterization of the contact material 602 as bone 608 and receiving a user input, such as a press of a button or depression of a foot pedal. In some implementations, the controller may continuously send the position of the surgical instrument 56 as the distal tip 412 of the surgical instrument 56 swept or traced across the anatomy and characterizes that the material of the anatomy in contact with the distal tip 412 along the sweeping or tracing path.

As described above with reference to the tracker 48 and localizer 34, upon the proper user input and characterization of a material type of the surgical object 600, the anatomy is registered within the surgical navigation system 20. Position information for the navigation system 20 is obtained using the surgical instrument 56 as the position of the distal tip 412 of the surgical instrument 56 is known with reference to the tracker 48. The position can then be calibrated to a known location in the field to determine the positions of the surgical object 600 to be contacted or avoided during a surgical procedure. As multiple points on the surgical object 600 are touched by the distal tip 412, the object can be mapped to define a position and orientation in the localizer coordinate system, and a model can be created. In some examples, the surgical instrument 56 may be dragged along the surface continuously to create a surface model representation of various tissue types. The created models may be used as virtual constraint boundaries to guide the movement of other instruments during a surgical procedure. The models may be displayed on the displays in addition to virtual representations of at least one of the anatomy, an instrument, a registration point, or the material characterization.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

## Claims

1. A surgical system (10) comprising:
a navigation system (20) comprising a localizer (34); and
a surgical instrument (56, 22) comprising:
a body (58);
a tracking device (48) coupled to the body (58) and being configured to be detected by the localizer (34) of the navigation system (20); and
a shaft (60) coupled to the body (58) and comprising a distal tip (412) configured to contact a material associated with an anatomy (TS);
a first conductor (402) extending along the shaft (60) to the distal tip (412); and
a second conductor (404) being insulated from the first conductor (402) and extending along the shaft (60) to the distal tip (412); and
one or more controllers (54, 26) coupled to the first and second conductors (402, 404) and being configured to:
command, across the first and second conductors (402, 404), transmission of a signal having a variable frequency;
based on the signal, measure an impedance of the material as the distal tip (412) is contacting the material; and
characterize a type of the material based on the measured impedance.

2. The surgical system (10) of claim 1, wherein:
the distal tip (412) of the surgical instrument (56, 22) is configured to contact the material to facilitate acquisition of registration points pursuant to an anatomical registration procedure involving registration of the anatomy (TS) to the localizer (34) of the navigation system (20); and
each registration point is based on a position of the distal tip (412) detected by the localizer (34).

3. The surgical system (10) of claim 2, wherein the one or more controllers (54, 26) are configured to evaluate the characterized material type to determine whether to acknowledge one or more of the registration points during the anatomical registration procedure.

4. The surgical system (10) of claim 3, wherein the one or more controllers (54, 26):
automatically acknowledge the one or more of the registration points in response to determining that the characterized material type is bone; or
acknowledge the one or more of the registration points in response to determining that the characterized material type is bone and in response to receipt of a user input to acknowledge the one or more of the registration points.

5. The surgical system (10) of claim 3, wherein:
the one or more controllers (54, 26) disregard the one or more of the registration points in response to determining that the characterized material type is non-bone material;
and optionally,
wherein the non-bone material comprises one or more of: cartilage, soft tissue, ligamentous tissue, tendon tissue, a blood vessel, gas or air adjacent to the anatomy (TS), metal material, implant material, a surgical tool material, and fluid material.

6. The surgical system (10) of claim 5, further comprising a feedback device (106), wherein the one or more controllers (54, 26) control the feedback device (106) to generate:
a first feedback in response to the characterized material type being bone; and
a second feedback in response to the characterized material type being non-bone material, wherein the second feedback is different from the first feedback;
and optionally;
wherein the one or more controllers (54, 26) control the feedback device (106) to generate the second feedback only if the non-bone material is cartilage.

7. The surgical system (10) of claim 6, wherein the one or more controllers (54, 26) control the feedback device (106) to generate a third feedback only if the non-bone material is other than cartilage, wherein the third feedback is different from the first and second feedback.

8. The surgical system (10) of any one of claims 2-8, wherein the navigation system (20) comprises a display (28) and the navigation system (20) is configured to present, on the display (28), a graphical representation of the anatomy (TS) and the surgical instrument (56, 22), a relative spatial relationship between the surgical instrument (56, 22) and the anatomy (TS) as determined by the localizer (34), the one or more of the registration points located on a surface of the of the graphical representation of the anatomy (TS), and an indication of the characterized material type associated with the one or more of the registration points.

9. The surgical system (10) of any preceding claim, wherein the one or more controllers (54, 26) comprise:
a first controller (54) fixed to the body (58) of the surgical instrument (56, 22) to:
command, across the first and second conductors (402, 404), transmission of the signal having the variable frequency; and
based on the signal, measure the impedance of the material as the distal tip (412) is contacting the material; and
a second controller (26) located remote from the surgical instrument (56, 22) to:
receive the measured impedance to characterize the type of the material.

10. The surgical system (10) of any preceding claim, wherein the variable frequency is implemented, by the one or more controllers (54, 26), as a frequency sweep across a specified range.

11. The surgical system (10) of claim 10, wherein the specified range is defined from 6kHz to 100 kHz;
and optionally;
wherein the one or more controllers (54, 26) perform the frequency sweep in response to detection of contact between the distal tip (412) and the material.

12. The surgical system (10) of any preceding claim, wherein:
the distal tip (412) integrally extends from the shaft (60);
the first conductor (402) terminates at a distal conductor end (430);
the distal tip (412) circumferentially surrounds the distal conductor end (430); and
the second conductor (404) is integrally coupled to the shaft (60) and terminates at the distal tip (412).

13. The surgical system (10) of any preceding claim, wherein the body (58) of the surgical instrument (56, 22) is configured to be freely held by a user.

14. The surgical system (10) of any one of claims 1-12, wherein:
the surgical instrument (56, 22) is coupled to a robotic arm (12) that is configured to actively move the surgical instrument (56, 22) relative to the anatomy (TS); or
the surgical instrument (56, 22) is coupled to a support arm that is configured to passively move the surgical instrument (56, 22) relative to the anatomy (TS) in response to user-guided movement.

15. A surgical instrument (56, 22) comprising:
a body (58);
a tracking device (48) coupled to the body (58) and being configured to be detected by a navigation system (20);
a shaft (60) coupled to the body (58) and comprising a distal tip (412) configured to contact a material associated with an anatomy (TS);
one or more controllers (54) coupled to the body (58);
a first conductor (402) coupled to the one or more controllers and extending along the shaft (60) to the distal tip (412); and
a second conductor (404) coupled to the one or more controllers, being insulated from the first conductor (402), and extending along the shaft (60) to the distal tip (412); and
wherein the one or more controllers (54) are configured to:
command, across the first and second conductors (402, 404), transmission of a signal having a variable frequency;
based on the signal, measure an impedance of the material as the distal tip (412) is contacting the material; and
characterize a type of the material based on the measured impedance.
